# EUROPÄISCHE PATENTSCHRIFT

(11) **EP 1 129 080 B1**
(45) Veröffentlichungstag und Bekanntmachung des Hinweises auf die Patenterteilung: **20.08.2003**
(21) Anmeldenummer: 99972208.5
(22) Anmeldetag: 05.11.1999
(51) Int. Cl.: C07D 251/62, C07D 251/60

(54) **VERFAHREN ZUR HERSTELLUNG VON REINEM MELAMIN**
METHOD FOR PRODUCING PURE MELAMINE
PROCEDE DE PRODUCTION DE MELAMINE PURE

(30) Priorität: 13.11.1998 AT 189498
(43) Veröffentlichungstag der Anmeldung: 05.09.2001
(73) Patentinhaber: Agrolinz Melamin GmbH, A-4021 Linz (AT)
(72) Erfinder: COUFAL, Gerhard, A-4060 Leonding (AT)
(74) Vertreter: Gross, Felix
(86) Internationale Anmeldenummer: EP9908462
(87) Internationale Veröffentlichungsnummer: WO00029393

(56) Entgegenhaltungen:
- WO-A-96/20182
- WO-A-96/23778
- WO-A-97/20826

## Beschreibung

Die Erfindung betrifft ein Verfahren zur Herstellung von reinem Melamin in einem Hochdruckprozeß, bei dem die Melaminschmelze vor der Verfestigung unter Zufuhr von Ammoniak gekühlt und anschließend wäßrig aufgearbeitet wird.
Die Herstellung von Melamin durch Pyrolyse von Harnstoff ist beispielsweise aus "Ullmann's Encyclopedia of Industrial Chemistry, Vol A16, 5^{th} ed (1990), Seiten 171-185" bekannt. In dem darin beispielhaft beschriebenen Montedison-Prozeß wird Harnstoff in einem Reaktor bei 370 °C und 70 bar gemeinsam mit Ammoniak während 20 min zersetzt. Das dabei im wesentlichen aus einer Melaminschmelze, Ammoniak und CO₂ bestehende Reaktionsgemisch wird anschließend in einen Quencher auf 25 bar entspannt und bei 160 °C mit einer wäßrigen NH₃/CO₂-Lösung behandelt, wobei festes Melamin ausfällt. Um eventuell nicht umgesetzten Harnstoff oder Nebenprodukte zu zersetzen, wird die dabei erhaltene Rohmelamin-Suspension gegebenenfalls für einige Zeit im Quencher belassen. Anschließend wird die Melaminsuspension gegebenenfalls in einem Stripper von NH₃ und CO₂ befreit, durch Zugabe von Mutterlauge verdünnt, wobei das Melamin gelöst wird. Nach Zugabe von Natronlauge und Aktivkohlebehandlung wird das Melamin auskristallisiert.

In einem weiteren Melaminprozeß (Nissan-Prozeß) erfolgt die Harnstoffzersetzung bei 100 bar und 400 °C, wobei die eingesetzte Harnstoffschmelze vor der Melaminsynthese zum Auswaschen von Melamin und Harnstoff aus den Offgasen des Melaminreaktors verwendet wird. Die erhaltene Melaminschmelze wird, gegebenenfalls nach einem "aging"-Schritt, mit einer wäßrigen Ammoniaklösung gequencht, dabei gelöst und zur Zersetzung von Verunreinigungen bei 180 °C verweilen gelassen. Nach Strippen des Ammoniaks und Filtrieren der Lösung wird das Melamin auskristallisiert. Gemäß US 3,637,686 wird die Melaminschmelze vor dem Quenchen mit wässrigem Ammoniak in einem ersten Schritt mit kaltem flüssigem oder gasförmigem Ammoniak bei einem Druck von 5 bis 100 bar und einer Temperatur von 200 bis 270 °C gequencht, wobei sich das Melamin verfestigt.

Das bei der Melaminsynthese primär anfallende Rohmelamin, das je nach Herstellverfahren ca. 94 bis 97 Gew.% Melamin, sowie insbesondere Melam, Melem, Melon, Ureidomelamin, Ammelin und Ammelid als wesentliche Verunreinigungen enthält, ist jedoch wegen der nicht ausreichenden Qualität der damit herstellbaren Harze für die meisten Anwendungen nicht bzw. nur unzureichend geeignet. Um zu einem reinen Melamin zu gelangen sind zusätzliche weitere Verfahrensschritte, wie z.B. Umkristallisieren, notwendig.

Es stellte sich demnach die Aufgabe, ein einfacheres Verfahren zu finden, mit dem Melamin in besserer Reinheit sowie mit guten Ausbeuten erhalten werden kann. Erfindungsgemäß konnte dieses Ziel dadurch erreicht werden, dass die vom Melaminreaktor kommende Rohmelaminschmelze vor der Verfestigung und wässrigen Aufarbeitung unter Aufnahme von weiterem Ammoniak bis nahe über den Schmelzpunkt abgekühlt wird.

Gegenstand der vorliegenden Erfindung ist demnach ein Verfahren zur Herstellung von reinem Melamin, dadurch gekennzeichnet, daß die in einem Hochdruckprozeß aus Harnstoff hergestellte Melaminschmelze, gegebenenfalls nach Strippen der Offgase, während 1 min bis 10 Stunden unter Zufuhr von kaltem, flüssigem, gasförmigem oder überkritischem NH₃ oder über Wärmeaustauscher oder durch eine Kombination dieser Kühlmaßnahmen auf eine Temperatur, die 1 bis 50 °C über dem vom jeweiligen Ammoniakdruck abhängigen Schmelzpunkt des Melamins liegt, gekühlt wird, 1 min bis 10 Stunden in diesem Temperaturbereich verweilen gelassen wird, worauf
a) durch Mischen mit Wasser oder einer wäßrigen, Ammoniak und/oder Melamin enthaltenden Lösung oder Suspension gequencht, das Melamin verfestigt und
b) das Melamin anschließend isoliert wird.

In Melamin-Hochdruckprozessen wird Melamin bei Drücken von etwa 70 bis 800 bar und Temperaturen von mindestens etwa 370 °C in flüssiger Form als Schmelze erhalten. Die bei der Melaminsynthese entstehenden, insbesondere NH₃ und CO₂ enthaltenden Offgase können entweder vor oder nach dem Abkühlen der Schmelze abgetrennt werden.
Vorteilhafterweise werden die Offgase durch Hindurchleiten durch eine Harnstoffschmelze gewaschen, wobei insbesondere mit den Offgasen mitgerissene Teilchen von Melamin bzw, von nicht umgesetztem Harnstoff ausgewaschen werden. Dabei wird die Harnstoffschmelze durch die heißen Offgase erwärmt und vorteilhafterweise zur Melaminsynthese in einen Melaminreaktor geführt, während die gereinigten Offgase vorteilhafterweise in einen Harnstoffreaktor geführt werden. Die Offgase können entweder direkt in den Harnstoffreaktor geführt werden, oder sie werden beispielsweise mit Hilfe von Ammoncarbonat- oder Ammoncarbamatlösungen, die beispielsweise in der Melaminanlage oder der Harnstoffanlage anfallen, kondensiert. Die dabei anfallende Wärme kann beispielsweise zum Vorwärmen des in der Harnstoffanlage eingesetzten Ammoniaks oder zur Produktion von Dampf verwendet werden.

Nach Abtrennen der Offgase kann die Melaminschmelze vorteilhafterweise gestrippt werden, beispielsweise mit NH₃, wodurch vor allem restliches CO₂ entfernt wird. Weiters ist es vorteilhaft, die Meiaminschmelze in einem Aging-Behälter verweilen zu lassen, wie beispielsweise in WO96/23778 oder WO96/20182 beschrieben. Die erfindungsgemäße Abkühlung auf die etwa 1 bis 50 °C über dem vom jeweiligen Ammoniakdruck abhängigen Schmelzpunkt des Melamins liegende Temperatur kann entweder über Wärmetauscher oder durch Zufuhr von kaltem flüssigem, gasförmigem oder überkritischem NH₃ in den Gasraum über der Schmelze erfolgen oder bevorzugt durch Einleiten in die Schmelze, bzw. durch eine Kombination dieser Kühlmaßnahmen, wobei eine gute Durchmischung, beispielsweise durch Durchleiten des NH₃, eventuell durch zusätzliche Mischeinrichtungen, wie z.B. Rührer, Statikmischer etc. von Vorteil ist. Dabei wird im Idealfall eine mit NH₃ gesättigte Melaminschmelze erhalten. in Abhängigkeit von den Verfahrensbedingungen sowie von der Menge des zugeführten NH₃ können erfindungsgemäß jedoch auch beim jeweiligen Druck und der jeweiligen Temperatur mit NH₃ übersättigte oder untersättigte Melaminschmelzen erhalten werden. Die Schmelze wird bevorzugt auf eine Temperatur abgekühlt, die etwa 1 bis 30 °C über dem vom jeweiligen Ammoniakdruck abhängigen Schmelzpunkt des Melamins liegt. Als besonders günstig erweist es sich, wenn auf eine solche Temperatur gekühlt wird, die möglichst knapp über dem vom jeweiligen Ammoniakdruck abhängigen Schmelzpunkt des Melamins liegt. Bevorzugt erfolgt die Abkühlung während einer Zeit von etwa 1 min bis 10 h, besonders bevorzugt von etwa 1 min bis 1 h. Die Verweilzeit in diesem Temperaturbereich beträgt etwa 1 min bis 10 h, bevorzugt etwa 1 min bis 1 h. Der Ammoniakdruck während der Abkühlung liegt bevorzugt bei etwa 50 bis 1000 bar, wobei Drücke von etwa 50 bis 400 bar besonders bevorzugt sind. Dabei ist es vorteilhaft, wenn durch die Ammoniakzufuhr gleichzeitig eine Druckerhöhung erfolgt.

Die Schmelzekühlung kann beispielsweise im Aging-Behälter oder auch in einem eigenen geeigneten Behälter oder Wärmetauscher erfolgen. Das im Anschluß an die Abkühlung der Schmelze erfolgende Quenchen erfolgt durch Vermischen der abgekühlten Melaminschmelze mit Wasser, einer wässrigen Lösung oder Suspension. Die Vermischung erfolgt besonders vorteilhaft und intensiv durch Versprühen oder Einleiten der einzelnen Substanzen, bzw. durch Versprühen oder Einleiten der Schmelze in die vorgelegte Lösung oder Suspension unter gleichzeitiger Druckminderung. Zum Quenchen der Melaminschmelze kann besonders vorteilhaft und wirtschaftlich die bei der Verfestigung bzw. Kristallisation und Isolierung des Melamins anfallende Mutterlauge rückgeführt und wieder verwendet werden. Die Temperatur beim Quenchen der Melaminschmelze mit Wasser oder einer wässrigen, Ammoniak und/oder Melamin enthaltenden Lösung oder Suspension liegt bevorzugt bei etwa 25 bis 300 °C, besonders bevorzugt bei etwa 50 bis 200 °C, der Druck bei etwa 1 bis 100 bar, besonders bevorzugt bei etwa 1 bis 50 bar. Die Temperatur stellt sich in Abhängigkeit von den Betriebsbedingungen im Quencher, insbesondere von Druck, Konzentrationen und Durchflußgeschwindigkeiten ein. Das bei der Abkühlung erhaltene kristallisierte bzw. verfestigte Melamin wird anschließend durch Abtrennen der Mutterlauge, beispielsweise durch Filtration oder Zentrifugieren isoliert und dann getrocknet, wobei Melamin vor allem wegen des Wegfalls weiterer Reinigungsschritte in guter Ausbeute und mit guter Reinheit im Bereich von etwa 99 % erhalten wird.

Das Quenchen erfolgt vorteilhaft kontinuierlich in einem der Schmelzekühlung nachgeschalteten Quench-Behälter. Dabei tritt beispielsweise flüssiges Melamin mit einer Temperatur, die knapp über dem vom jeweiligen Ammoniakdruck abhängigen Schmelzpunkt bis etwa 370 °C liegt und einem Druck von etwa 100 bis 400 bar am oberen Teil in den Quench-Behälter ein und wird sowohl mit Wasser als auch mit der im Kreislauf rückgeführten wässrigen Suspension von festem Melamin in einer wässrigen Melaminlösung aus dem Quench-Behälter bzw. mit rückgeführter Mutterlauge, die ebenfalls oben in den Quench-Behälter eingesprüht werden, gequencht. Das eingesprühte Wasser besitzt beispielsweise eine Temperatur von etwa 25 bis 90 °C, die eingesprühte Suspension bzw. Mutterlauge eine Temperatur von etwa 25 bis 150 °C bei einem Druck von etwa 1 bis 10 bar. Gemäß diesem Beispiel wird die Temperatur im Quench-Behälter etwa konstant gehalten, die Melamin-Suspension im Quench-Behälter wird vorteilhafterweise gerührt, der nicht im Kreislauf rückgeführte Teil der Melamin-Suspension, die neben festem Melamin auch gelöstes Melamin und Ammoniak enthält, wird kontinuierlich am unteren Teil des Quench-Behälters abgezogen, das Melamin durch Filtration oder Zentrifugieren isoliert und getrocknet und die Mutterlauge teils rückgeführt, teils ausgeschleust.
Zur Erzielung höherer und höchster Reinheitsgrade kann das Melamin umkristallisiert werden. Dabei ist es auch möglich, das gemäß a) erhaltene Melamin, ohne vorherige Isolierung, direkt in der Suspension durch Zufuhr einer wässrigen ammoniakalischen Lösung zu lösen, wobei besonders bevorzugt und wirtschaftlich die bei der Verfestigung bzw. Kristallisation des Melamins anfallende Mutterlauge rückgeführt und zum Lösen verwendet wird. Eventuell durch Hydrolyse gebildete Nebenprodukte, vor allem die Oxyaminotriazine, wie z.B. Ammelin und Ammelid, können gegebenenfalls durch Zugabe von Alkali, beispielsweise NaOH, in Lösung gehalten werden. Die Lösung wird gegebenenfalls verweilen gelassen, gegebenenfalls wird noch gelöstes NH₃ gestrippt und gegebenenfalls mit Aktivkohle behandelt. Anschließend wird filtriert, das Melamin auskristallisiert, beispielsweise durch weitere Abkühlung und/oder Verminderung des Druckes bzw. Anlegen eines Vakuums, von der Mutterlauge abgetrennt und getrocknet.
Das gemäß vorliegender Erfindung erhaltene Melamin besitzt im Vergleich zu herkömmlichem Melamin nach Quenchen mit Wasser eine höhere Reinheit, und die Ausbeute nach dem Umkristallisieren ist höher.
Im Anschluß an das Trocknen kann das Melamin zur weiteren Verbesserung der Qualität getempert werden. Das isolierte, gegebenenfalls umkristallisierte Melamin wird dabei bevorzugt unter NH₃-Druck von etwa 5 bis 600 bar, bevorzugt von etwa 5 bis 100 bar und bei einer Temperatur von etwa 100 °C bis unterhalb des vom jeweiligen NH₃-Druck abhängigen Schmelzpunktes des Melamins für eine Zeit von etwa 5 min bis 10 h, bevorzugt von etwa 5 min bis 5 h verweilen gelassen (getempert).

### Beispiel 1

In einen Autoklaven mit 100 ml Volumen wurden 20 g Melamin mit einem Melamgehalt von 2 Gew. % und einem Melemgehalt von 1 Gew. % eingebracht, der Autoklav unter NH₃ Zufuhr auf eine Temperatur von 370 °C bei einem NH₃-Druck von 250 bar gebracht und 2 h bei dieser Temperatur und diesem Druck gehalten. Dann wurde unter NH₃-Zufuhr während 1 h auf 320 °C abgekühlt, wobei der Druck von 250 bar gehalten wurde, 30 min bei dieser Temperatur gehalten und die Melaminschmelze anschließend in einen zweiten Autoklaven (1000 ml Volumen), in dem sich eine wäßrige Ammoniaklösung mit einer Temperatur von 159 °C bei einem Druck von 12 bar befanden, gesprüht. Dabei verfestigte sich das Melamin, die Temperatur im zweiten Autoklaven stieg auf 168 °C, der Druck auf 24 bar. Nach Abkühlen des Autoklaven, Filtrieren und Trocknen wurde Melamin mit einer Reinheit von 99,1 % erhalten.

### Beispiel 2

In einen Autoklaven mit 100 ml Volumen wurden 20 g Melamin mit einem Melamgehalt von 2 Gew. % und einem Melemgehalt von 1 Gew. % eingebracht, der Autoklav unter NH₃-Zufuhr auf eine Temperatur von 370 °C bei einem NH₃-Druck von 250 bar gebracht und 2 h bei dieser Temperatur und diesem Druck gehalten. Dann wurde unter NH₃-Zufuhr während 1 h auf 330 °C abgekühlt, wobei der Druck von 250 bar gehalten wurde, 30 min bei dieser Temperatur gehalten und die Melaminschmelze anschließend in einen zweiten Autoklaven (1000 ml Volumen), in dem sich eine wäßrige Ammoniaklösung mit einer Temperatur von 62 °C bei einem Druck von 1 bar befanden, gesprüht. Dabei verfestigte sich das Melamin, die Temperatur im zweiten Autoklaven stieg auf 81 °C, der Druck auf 4 bar. Nach Abkühlen des Autoklaven, Filtrieren und Trocknen wurde Melamin mit einer Reinheit von 98,8 % erhalten.

### Beispiel 3

In einen Autoklaven mit 100 ml Volumen wurden 20 g Melamin mit einem Melamgehalt von 2 Gew. % und einem Melemgehalt von 1 Gew. % eingebracht, der Autoklav unter NH₃-Zufuhr auf eine Temperatur von 370 °C bei einem NH₃-Druck von 250 bar gebracht und 2 h bei dieser Temperatur und diesem Druck gehalten. Dann wurde unter NH₃-Zufuhr während 1 h auf 350 °C abgekühlt, wobei der Druck von 250 bar gehalten wurde, 30 min bei dieser Temperatur gehalten und die Melaminschmelze anschließend in einen zweiten Autoklaven (1000 ml Volumen), in dem sich eine wäßrige Ammoniaklösung mit einer Temperatur von 63 °C bei einem Druck von 1 bar befanden, gesprüht. Dabei verfestigte sich das Melamin, die Temperatur im zweiten Autoklaven stieg auf 88 °C, der Druck auf 4 bar. Nach Abkühlen des Autoklaven, Filtrieren und Trocknen wurde Melamin mit einer Reinheit von 98,6 % erhalten.

### Vergleichsbeispiel 1

In einen Autoklaven mit 100 ml Volumen wurden 20 g Melamin mit einem Melamgehalt von 2 Gew. % und einem Melemgehalt von 1 Gew. % eingebracht, der Autoklav unter NH₃-Zufuhr auf eine Temperatur von 370 °C bei einem NH₃-Druck von 250 bar gebracht und 2 h bei dieser Temperatur und diesem Druck gehalten. Dann wurde die Melaminschmelze in einen zweiten Autoklaven (1000 ml Volumen), in dem sich eine wäßrige Ammoniaklösung mit einer Temperatur von 62 °C bei einem Druck von 1 bar befanden, gesprüht. Dabei verfestigte sich das Melamin, die Temperatur im zweiten Autoklauen stieg auf 94 °C, der Druck auf 5 bar. Nach Abkühlen des Autoklaven, Filtrieren und Trocknen wurde Melamin mit einer Reinheit von 97,5 % erhalten.

## Patentansprüche

1. Verfahren zur Herstellung von reinem Melamin, **dadurch gekennzeichnet, daß** die in einem Hochdruckprozeß aus Harnstoff hergestellte Melaminschmelze, gegebenenfalls nach Strippen der Offgase, während 1 min bis 10 Stunden unter Zufuhr von kaltem, flüssigem, gasförmigem oder überkritischem NH₃ oder über Wärmeaustauscher oder durch eine Kombination dieser Kühlmaßnahmen auf eine Temperatur, die 1 bis 50 °C über dem vom jeweiligen Ammoniakdruck abhängigen Schmelzpunkt des Melamins liegt, gekühlt wird, 1 min bis 10 Stunden in diesem Temperaturbereich verweilen gelassen wird, worauf
a) durch Mischen mit Wasser oder einer wäßrigen, Ammoniak und/oder Melamin enthaltenden Lösung oder Suspension gequencht, das Melamin verfestigt und
b) das Melamin anschließend isoliert wird.

2. Verfahren gemäß Anspruch 1, **dadurch gekennzeichnet, daß** die Abkühlung der Melaminschmelze auf die Temperatur, die 1 bis 50 °C über dem Schmelzpunkt des Melamins liegt, durch Einleiten von kaltem flüssigem oder gasförmigem Ammoniak erfolgt.

3. Verfahren gemäß Anspruch 1, **dadurch gekennzeichnet, dass** die Melaminschmelze auf eine Temperatur, die 1 bis 30 °C über dem Schmelzpunkt des Melamins liegt, abgekühlt wird.

4. Verfahren gemäß Anspruch 1, **dadurch gekennzeichnet, dass** die Melaminschmelze bei einem Ammoniakdruck von 50 bis 400 bar unter Zufuhr von Ammoniak auf eine Temperatur, die 1 bis 50 °C über dem Schmelzpunkt des Melamins liegt, abgekühlt wird.

5. Verfahren gemäß Anspruch 1, **dadurch gekennzeichnet, dass** die Melaminschmelze in Stufe a) durch rückgeführte, bei der Kristallisation anfallende Mutterlauge gequencht wird.

6. Verfahren gemäß Anspruch 1, **dadurch gekennzeichnet, dass** das gemäß Stufe
a) erhaltene, als Suspension vorliegende Melamin, durch Zufuhr einer wässrigen ammoniakalischen Lösung, bevorzugt einer rückgeführten bei der Kristallisation anfallenden Mutterlauge gelöst, die Lösung gegebenenfalls mit NaOH versetzt, gegebenenfalls verweilen gelassen, gegebenenfalls das gelöste Ammoniak gestrippt, anschließend filtriert und das Melamin auskristallisiert und isoliert wird.

7. Verfahren gemäß Anspruch 1, **dadurch gekennzeichnet, dass** in Stufe a) bei einer Temperatur von 25 °C bis 300 °C und einem Druck von 1 bis 100 bar gequencht wird.

8. Verfahren gemäß Anspruch 1, **dadurch gekennzeichnet, dass** in Stufe a) bei einer Temperatur von 50 - 200 °C gequencht wird.

9. Verfahren gemäß Anspruch 1, **dadurch gekennzeichnet, dass** in Stufe a) bei einem Druck von 1 - 50 bar gequencht wird.

## Claims

1. Process for the production of pure melamine, **characterised in that** the melamine melt produced from urea in a high pressure process, optionally after stripping of the off gases, is cooled for 1 min to 10 hours with the introduction of cold, liquid, gaseous or super-critical NH₃ or by heat exchangers or by a combination of these cooling measures to a temperature which lies 1 to 50°C above the melting point of the melamine, which is dependent on the particular ammonia pressure, is allowed to stand in this temperature range for 1 min to 10 hours, whereupon
a) quenched by mixing with water or an aqueous solution or suspension containing ammonia and/or melamine, the melamine is solidified and
b) the melamine is then isolated.

2. Process according to Claim 1, **characterised in that** the cooling of the melamine melt to the temperature which lies 1 to 50°C above the melting point of the melamine is effected by introduction of cold liquid or gaseous ammonia.

3. Process according to Claim 1, **characterised in that** the melamine melt is cooled to a temperature which lies 1 to 30°C above the melting point of the melamine.

4. Process according to Claim 1, **characterised in that** the melamine melt is cooled under an ammonia pressure of 50 to 400 bars with introduction of ammonia to a temperature which lies 1 to 50°C above the melting point of the melamine.

5. Process according to Claim 1, **characterised in that** the melamine melt is quenched in step a) with recycled mother liquor arising in the crystallisation.

6. Process according to Claim 1, **characterised in that** the melamine obtained according to step a), present as a suspension, is dissolved by addition of an aqueous ammoniacal solution, preferably of a recycled mother liquor arising in the crystallisation, the solution is optionally treated with NaOH, optionally allowed to stand, the dissolved ammonia is optionally stripped, and then filtered, and the melamine crystallised out and isolated.

7. Process according to Claim 1, **characterised in that** in step a) quenching is performed at a temperature of 25°C to 300°C and a pressure of 1 to 100 bars.

8. Process according to Claim 1, **characterised in that** in step a) quenching is performed at a temperature of 50-200°C.

9. Process according to Claim 1, **characterised in that** in step a) quenching is performed at a pressure of 1-50 bars.

## Revendications

1. Procédé de préparation de mélamine pure, **caractérisé en ce que** l'on refroidit la masse fondue de mélamine préparée à partir d'urée dans un procédé haute pression, éventuellement après avoir éliminé les effluents gazeux, pendant 1 minute à 10 heures en amenant du NH₃ froid, liquide, gazeux ou supercritique ou par l'intermédiaire d'échangeurs de chaleur ou par une combinaison de ces mesures de refroidissement, à une température qui se situe de 1 à 50°C au-dessus du point de fusion de la mélamine qui dépend de la pression d'ammoniac correspondante, on la laisse séjourner pendant 1 minute à 10 heures dans ce domaine de température, après quoi
a) on refroidit rapidement en mélangeant avec de l'eau ou une solution ou suspension aqueuse contenant de l'ammoniac et/ou de la mélamine, la mélamine se solidifie et
b) on isole ensuite la mélamine.

2. Procédé selon la revendication 1, **caractérisé en ce que** le refroidissement de le la masse fondue de mélamine à la température se situant de 1 à 50°C au-dessus du point de fusion de la mélamine s'effectue par introduction d'ammoniac liquide ou gazeux froid.

3. Procédé selon la revendication 1, **caractérisé en ce que** l'on refroidit la masse fondue de mélamine à une température se situant de 1 à 30°C au-dessus du point de fusion de la mélamine.

4. Procédé selon la revendication 1, **caractérisé en ce que** l'on refroidit la masse fondue de mélamine sous une pression d'ammoniac de 50 à 400 bar en amenant de l'ammoniac à une température se situant de 1 à 50°C au-dessus du point de fusion de la mélamine.

5. Procédé selon la revendication 1, **caractérisé en ce que** l'on refroidit rapidement la masse fondue de mélamine dans l'étape a) avec de la liqueur mère recyclée formée lors de la cristallisation.

6. Procédé selon la revendication 1, **caractérisé en ce que** l'on dissout la mélamine obtenue dans l'étape a) et se trouvant sous forme de suspension en introduisant une solution aqueuse ammoniacale, de préférence une liqueur mère formée lors de la cristallisation et recyclée, on ajoute éventuellement du NaOH à la solution, on laisse éventuellement reposer, on élimine éventuellement l'ammoniac dissous, puis on filtre, on cristallise et on isole la mélamine.

7. Procédé selon la revendication 1, **caractérisé en ce que**, dans l'étape a), on refroidit rapidement à une température de 25°C à 300°C et sous une pression de 1 à 100 bar.

8. Procédé selon la revendication 1, **caractérisé en ce que**, dans l'étape a), on refroidit rapidement à une température de 50 à 200°C.

9. Procédé selon la revendication 1, **caractérisé en ce que**, dans l'étape a), on refroidit rapidement sous une pression de 1 à 50 bar.
